(19) Europäisches Patentamt
European Patent Office
Office européen des brevets

(11) Publication number: **0 613 886 A1**

(12) **EUROPEAN PATENT APPLICATION**

(21) Application number: **94200495.3**

(22) Date of filing: **25.02.94**

(51) Int. Cl.5: **C07D 209/76**, C10M 133/16, C10M 133/56, C10L 1/22

(30) Priority: **01.03.93 EP 93301551**

(43) Date of publication of application: **07.09.94 Bulletin 94/36**

(84) Designated Contracting States: **BE DE ES FR GB IT NL**

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Scott, Richard Mark**
**Hamlet Court,**
**Heart's Delight**
**Borden, Sittingbourne, Kent ME9 8HX (GB)**
Inventor: **Shaw, Robert William**
**89 Gadby Road**
**Sittingbourne, Kent ME10 1PY (GB)**

(54) **Cyclopentadiene derivatives as dispersants for lubricating vils and for fuels.**

(57) The invention provides compounds of the general formula

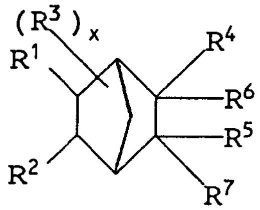

(I)

in which $R^1$ and $R^2$ each represent a hydrogen atom, or together represent a carbon-carbon single bond; each of $R^4$ and $R^5$ independently represents a hydrogen atom, or a $C_1$-$C_{20}$ alkyl or phenyl group, each of which may be optionally substituted; $R^6$ represents a hydrogen atom, or a $C_1$-$C_{20}$ alkyl or phenyl group, each of which may be optionally substituted and $R^7$ represents a group $-CH_2-NHR^8$ in which $R^8$ represents an optionally substituted alkyl group, or a group -COX wherein X represents an optionally substituted alkoxy group or $-NHR^8$ where $R^8$ is as defined above; or $R^6$ and $R^7$ together represent a group

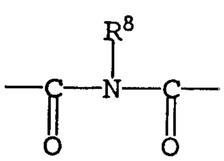 , 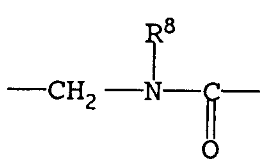 , or 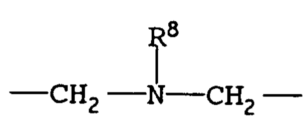

where $R^8$ is as defined above; x is 1 to 6; and each $R^3$ independently represents an optionally substituted alkyl or alkenyl group or a group of formula

EP 0 613 886 A1

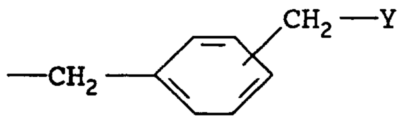

(II) ,

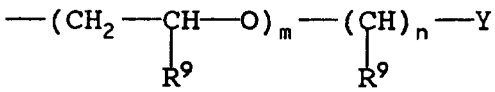

(IIIA)

or

—D—Y (IIIB)

wherein m is in the range 0 to 25, n is in the range 1 to 20, each $R^9$ independently represents a hydrogen atom or a methyl or ethyl group, D is derived from a saturated or unsaturated hydrocarbon containing from 20 to 500 carbon atoms, and Y represents a group of formula

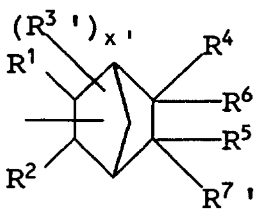

(IV)

in which $R^1$, $R^2$, $R^4$, $R^5$ and $R^6$ are as defined above; x' is 0 to 5; $R^{3'}$ is as defined for $R^3$ other than a group of formula II, IIIA or IIIB, $R^{7'}$ represents a group $R^7$ as defined above or a group -CHO or -COOH, or $R^6$ and $R^{7'}$ together represent a group

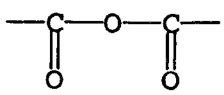

,

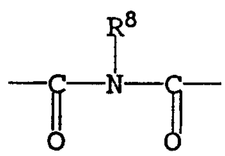

,

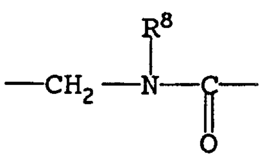

or

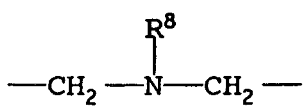

where $R^8$ is as defined above, each group $R^3$ containing a total of up to 550 carbon atoms, with the provisos that up to a maximum of 3 $R^3$ groups can each simultaneously contain a total of greater than 40 carbon atoms and that the groups $R^3$ taken together contain a total of greater than 20 carbon atoms; a process for their preparation; novel intermediates; lubricating oil compositions, fuel compositions and additive concentrates containing the formula I compounds; and the use of the formula I compounds as dispersant additives.

The present invention relates to cyclopentadiene derivatives, a process for their preparation, lubricating compositions, fuel compositions and additive concentrates containing them, their use as dispersant additives, and novel intermediates.

U.S. Patent No. 3,311,634 discloses 7-Y-N-$R_3$-($R_4$-5-norbornene)-2,3-dicarboximides and the corresponding norbornanes having the formulae

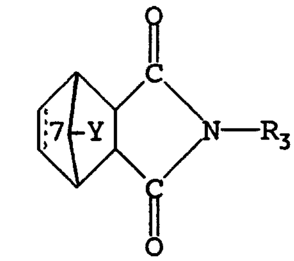

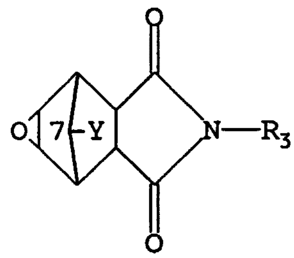

wherein Y is a member selected from the group consisting of

$R_1$,$R_2$ - methylene

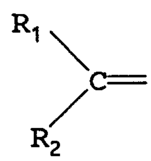

,

$R_1$,$R_2$ - spirooxirane

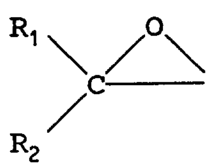

,

$R_1$,$R_2$ - methyl

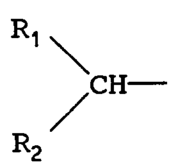

,

and

$R_1$,$R_2$ - methyl

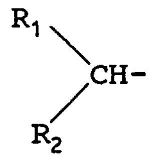 .

wherein the 7-carbon of the norbornene(ane) is further substituted with hydroxyl.

Although none of the above formulae shows any group "$R_4$", at column 1, lines 40 to 45 it is stated:- "The $R_3$ substituents, other than hydrogen, are attached to the imide nitrogens by means of a single carbon-nitrogen bond and the $R_4$ substituents, when other than hydrogen, are attached to one or more of the available carbons on the ring nucleus.".

$R_1$ and $R_2$ can be the same or different substituted or unsubstituted carbocyclic groups. $R_1$ and $R_2$ can further be heterocyclic groups containing from 4 to 5 carbon atoms interrupted by oxygen, nitrogen or sulphur linkages. $R_2$ can also be a saturated or unsaturated, substituted or unsubstituted, aliphatic group containing 1 to 7 carbon atoms.

$R_3$ can be hydrogen, a lower alkyl, carbamoyl or hydroxyloweralkyl group, a heterocyclic group containing from 4 to 5 carbon atoms interrupted by oxygen, nitrogen or sulphur linkages, or an aralkyl, aralkenyl, aryl, substituted aryl carbonylloweralkyl, aminoloweralkyl or loweralkylaminoloweralkyl group.

$R_4$ can be any one or more of the substituents defined for $R_1$, $R_2$ and $R_3$ including hydrogen. $R_4$ can also be a lower alkenyl, lower alkynyl, cycloalkyl or cycloalkylloweralkyl group, optionally substituted with nitro, amino or hydroxyl groups or halogen atoms. It is stated at column 2, lines 25 to 28:- "Amino and hydroxyl substituents may be linked to the lower hydrocarbon group (represented by $R_4$) or they may be attached directly to the ring, i.e. they may be $R_4$." $R_4$ may also be an oxygen atom attached through single bonds to each of the 5- and 6-positions of the norbornene-(ane) nucleus, i.e. an epoxy group.

Although there are examples in the patent specification where one $R_4$ group is present, e.g. Examples XLVIII and LI, in each case the $R_4$ group is either an epoxy or hydroxyl group. In addition, the only Y groups exemplified contain at most a total of 14 carbon atoms, e.g. an o-methyldiphenylmethylene group as shown in Example XXXII.

The compounds of the above formulae are said to be useful as intermediates in the preparation of 2-$R_3$-3a,4,7,7a-tetrahydro-4,7-(Y)$R_4$-isoindolines, the corresponding 3a, 4, 5, 6, 7, 7a-hexahydro compounds, N-oxides and acid addition salts thereof, and quarternary ammonium compounds, which are therapeutically active compounds useful in the treatment of peptic ulcers. Those compounds (intermediates) in which $R_1$ and/or $R_2$ is/are aromatic and Y is an $R_1$, $R_2$-methylene function are said to be useful as ultraviolet absorbers, whilst certain compounds (intermediates) are said to possess CNS depressant activity or anti-inflammatory activity.

U.S. Patent No. 3,320,267 discloses 7-$R_1$, $R_2$-Y-2-$R_3$-3-$R_4$-norbornenes, the corresponding norbornanes and their salts. The $R_1$, $R_2$-bearing Y is a saturated or unsaturated aliphatic group containing from 2 to 7 carbon atoms, one of which comprises the 7-position carbon atom of the norbornene/norbornane nucleus, which may be substituted with a hydroxy or epoxy group.

$R_1$ and $R_2$ can be the same or different, substituted or unsubstituted aromatic lower carbocyclic aryl groups. $R_1$ and $R_2$ can also be a substituted or unsubstituted lower alkyl group, or a heterocyclic group containing from 4 to 5 carbon atoms interrupted by oxygen, nitrogen or sulphur linkages.

The only $R_1$, $R_2$-bearing Y groups which are exemplified contain at most a total of 16 carbon atoms as in Example XLIV.

$R_3$ can be hydrogen, or a carboxamido, substituted carboxamido, amino, aminomethyl, substituted aminomethyl, carboxyl, esterified carboxyl or hydroxymethyl group.

There is no definition as such for $R_4$ in the patent but it is apparent (from column 2, lines 4 to 6, 54 and 55) that it can represent a substituted or unsubstituted lower hydrocarbon group, a carboxyl group, or an esterified carboxyl group.

It is indicated from column 1, line 63 to column 2, line 18 that compounds of the above formula and the corresponding norbornanes may be further substituted at any one of the available positions in the ring nucleus by any one or more substituents defined for $R_1$, $R_2$, $R_3$ and $R_4$ or by a lower alkynyl, cycloalkyl, cycloalkylloweralkyl, or amino group to produce compounds of formula 7-$R_1$, $R_2$-Y-2-$R_3$-5-norbornene-$R_5$-3-$R_4$ and the corresponding norbornanes in which $R_5$ represents the substituent further included in the ring. It would appear from Example LIV that $R_5$ may also represent an oxygen atom attached through single bonds to each of the 5- and 6-positions of the norbornene-(ane) nucleus, i.e. $R_5$ is an epoxy group. These are no other examples where an $R_5$ substituent is present.

The compounds of the above formulae, especially those in which the $R_3$ and $R_4$ substituents are carboxamido and carboxyl groups respectively, are said to be useful as intermediates in the preparation of 7-$R_1$,$R_2$-Y-N-$R_3$-5-norbornene-$R_4$-2,3-dicarboximides and the corresponding norbornanes, which in turn are useful as intermediates in the preparation of 2-$R_3$-3a,4,7,7a-tetrahydro-4,7-($R_1$,$R_2$-Y)-$R_4$-isoindolines, the corresponding 3a,4,5,6,7,7a-hexahydro compounds, N-oxides and acid addition salts thereof, and quaternary ammonium compounds which are therapeutically active compounds useful in the treatment of peptic ulcers.

U.S. Patent No. 3,641,108 discloses propargyl esters of the general formula

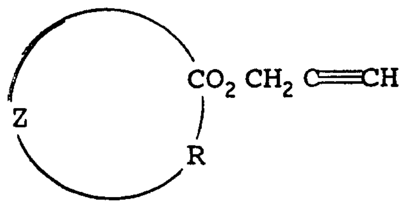

wherein R is hydrogen or alkyl and Z is a 1,3 - cyclopentylene or 1,3 - cyclopent-4-enylene group having no substituents other than lower alkyl, there being no more than 5, and preferably no more than 1, of such substituents if any is present. By the term lower alkyl is meant linear or branched chain alkyl of up to about 4 carbons, such as methyl, ethyl, isopropyl, tert-butyl and the like, with methyl being preferred.

The propargyl esters are said to be useful as bactericides and fungicides.

It has now surprisingly been found that certain cyclopentadiene derivatives possess useful dispersant properties.

In accordance with the present invention, there is provided a compound of the general formula

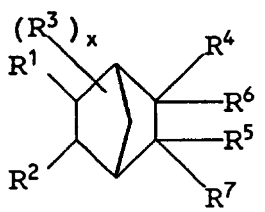

(I)

in which $R^1$ and $R^2$ each represent a hydrogen atom, or together represent a carbon-carbon single bond; each of $R^4$ and $R^5$ independently represents a hydrogen atom, or a $C_1$-$C_{20}$ alkyl or phenyl group, each of which may be optionally substituted; $R^6$ represents a hydrogen atom, or a $C_1$-$C_{20}$ alkyl or phenyl group, each of which may be optionally substituted and $R^7$ represents a group $-CH_2-NHR^8$ in which $R^8$ represents an optionally substituted alkyl group, or a group -COX wherein X represents an optionally substituted alkoxy group or $-NHR^8$ where $R^8$ is as defined above; or $R^6$ and $R^7$ together represent a group

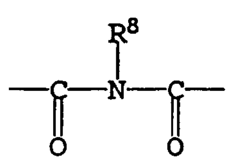

,

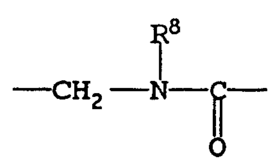

, or

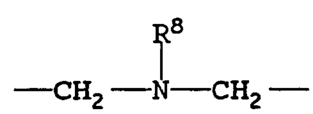

where $R^8$ is as defined above; x is 1 to 6, preferably 1 to 4; and each $R^3$ independently represents an optionally substituted alkyl or alkenyl group or a group of formula

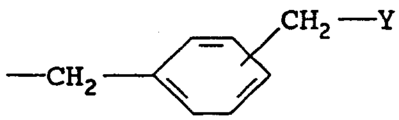

(II) ,

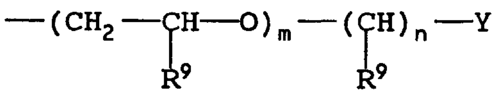

(IIIA)

or

—D—Y (IIIB)

wherein m is in the range 0 to 25, n is in the range 1 to 20, each $R^9$ independently represents a hydrogen atom or a methyl or ethyl group, D is derived from a saturated hydrocarbon containing from 20 to 500 carbon atoms, and Y represents a group of formula

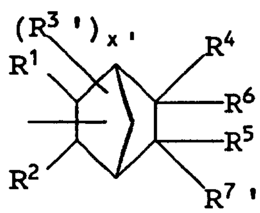

(IV)

in which $R^1$, $R^2$, $R^4$, $R^5$ and $R^6$ are as defined above; x' is 0 to 5; $R^{3'}$ is as defined for $R^3$ other than a group of formula II, IIIA or IIIB, $R^{7'}$ represents a group $R^7$ as defined above or a group -CHO or -COOH, or $R^6$ and $R^{7'}$ together represent a group

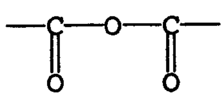 , 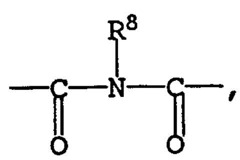 ,

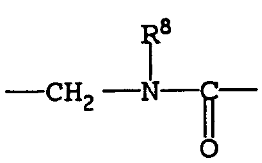 or 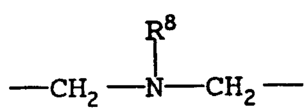

where $R^8$ is as defined above, each group $R^3$ containing a total of up to 550 carbon atoms, with the provisos that up to a maximum of 3 $R^3$ groups can each simultaneously contain a total of greater than 40 carbon atoms and that the groups $R^3$ taken together contain a total of greater than 20 carbon atoms. Thus, when one $R^3$ group contains more than 40 carbon atoms, not more than two other $R^3$ groups individually contain more than 40 carbon atoms.

In this specification, unless otherwise stated, an alkyl or alkenyl group or an alkyl moiety in an alkoxy or haloalkyl group may be linear or branched.

It is preferred that in formula I above $R^1$ and $R^2$ together represent a carbon-carbon single bond.

Each of $R^4$ and $R^5$ independently represents a hydrogen atom, or a $C_1$-$C_{20}$ alkyl or phenyl group, each of which may be optionally substituted. Examples of suitable substituent groups include halogen atoms and,

in relation to the phenyl group, additionally $C_1$-$C_6$ alkyl, $C_1$-$C_6$ alkoxy and $C_1$-$C_6$ haloalkyl groups.

Preferably each of $R^4$ and $R^5$ independently represents a hydrogen atom, a $C_1$-$C_{12}$ alkyl group, or a phenyl group optionally substituted by one or more substituents selected from $C_1$-$C_3$ alkyl, $C_1$-$C_3$ alkoxy and $C_1$-$C_3$ haloalkyl groups.

More preferably, each of $R^4$ and $R^5$ independently represents a hydrogen atom, a $C_1$-$C_6$ alkyl, especially methyl, group, or a phenyl group optionally substituted by one or two substituents selected from methyl and methoxy groups.

Most preferably, $R^4$ and $R^5$ both represent a hydrogen atom.

$R^6$ may represent a hydrogen atom, or a $C_1$-$C_{20}$ alkyl or phenyl group, each of which may be optionally substituted. Examples of suitable substituent groups are those mentioned above for $R^4$ and $R^5$.

Preferably $R^6$ represents a hydrogen atom, a $C_1$-$C_{12}$ alkyl group, or a phenyl group optionally substituted by one or more substituents selected from $C_1$-$C_3$ alkyl, $C_1$-$C_3$ alkoxy and $C_1$-$C_3$ haloalkyl groups.

More preferably, $R^6$ represents a hydrogen atom, $C_1$-$C_6$ alkyl, especially methyl, group, or a phenyl group optionally substituted by one or two substituents selected from methyl and methoxy groups.

Most preferably, $R^6$ represents a hydrogen atom.

$R^7$ may represent a group $-CH_2NHR^8$ in which $R^8$ represents an optionally substituted alkyl group, or a group -COX wherein X represents an optionally substituted alkoxy group or $-NHR^8$ where $R^8$ is as defined above.

When X represents an optionally substituted alkoxy group, it may be an unsubstituted $C_1$-$C_{20}$, preferably $C_{12}$-$C_{15}$, alkoxy group, or it may be a substituted alkoxy group such as a pentaerythritolderived moiety or a polyalkylene glycol-derived moiety, e.g. a group of formula $R^{10}$-O-$(CH_2CH_2O)_p$-, where p is 3 to 9 and $R^{10}$ is a $C_9$-$C_{15}$ alkyl group, or a group of formula HO-$(CH_2CH_2O)_q$-, where q is 4 to 14. Two groups X can together be a bridging group, e.g. a group

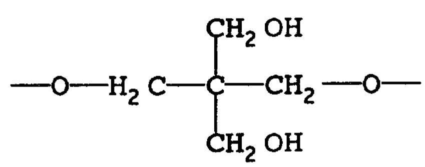

or a group of formula $—O—(CH_2CH_2O)_q—$ ,
where q is as defined above.

The groups $-NHR^8$ and $-NR^8$ are derived from primary amines containing one or more nitrogen atoms, e.g. 1 to 8 nitrogen atoms and, optionally, one or more oxygen atoms, e.g. 1 to 200 oxygen atoms.

For example, the primary amine may be a branched or unbranched, saturated, aliphatic $C_1$-$C_{18}$, preferably $C_1$-$C_{10}$, amine containing 1 or 2 nitrogen atoms such as methylamine, ethylamine, propylamine, butylamine, sec. butylamine, 1,2-diaminoethane, ethanolamine and 3-dimethylamino-1-propylamine. The primary amine may also be a compound of the general formula

$H_2N$-$(CHR^{11})_r$-$CH_2$-[A-$CH_2$-$(CHR^{11})_r]_s$-$NH_2$ (V)

wherein A is -NH or -O-, each $R^{11}$ independently represents a hydrogen atom or a methyl group, r is in the range 1 to 3, and s is in the range 1 to 8 when A is -NH or s is in the range 1 to 200 when A is -O-.

Particularly preferred compounds of formula V are those in which A is -NH, r is 1, each $R^{11}$ represents a hydrogen atom, and s is in the range 1 to 5; or A is -O-, r is 1, each $R^{11}$ represents a methyl group, and s is in the range 4 to 40.

The primary amine may further be a compound of the general formula

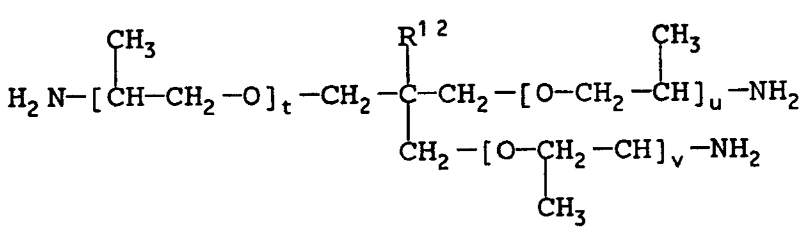
(VI)

in which $R^{12}$ represents a hydrogen atom or an ethyl group, wherein when $R^{12}$ represents a hydrogen atom, then each of t, u, and v is in the range 1 to 100 and when $R^{12}$ represents an ethyl group, then each of t, u and v is in the range 1 to 10.

Two groups $R^8$ can together be a bridging group, e.g. a group of formula

$-(CHR^{11})_r-CH_2-[A-CH_2-(CHR^{11})_r]_s-$ (V')

where A, $R^{11}$, r and s are as defined above, or a group of formula

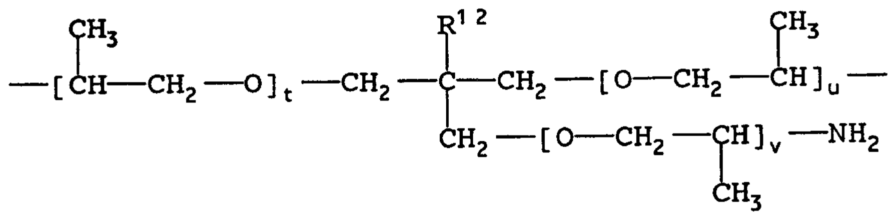

(VI')

where $R^{12}$, t, u and v are as defined above; or three groups $R^8$ can together be a bridging group of formula

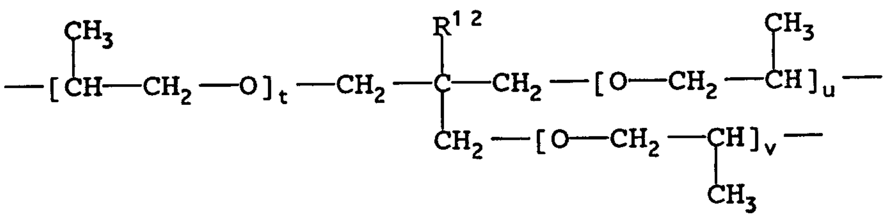

(VI'')

where $R^{12}$, t, u and v are as defined above.

$R^6$ and $R^7$ can together represent a group

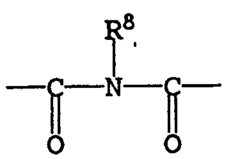 , 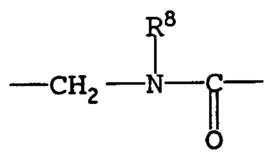 , or 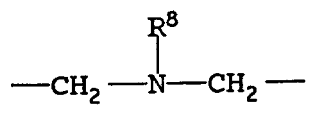

the first of these being particularly preferred.

It is preferred in formula I that each of $R^4$, $R^5$ and $R^6$ represents a hydrogen atom and $R^7$ represents a group $-CH_2NHR^8$, or that each of $R^4$ and $R^5$ represents a hydrogen atom and $R^6$ and $R^7$ together represent a group

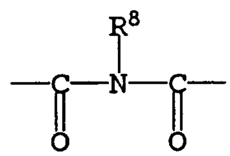

where $R^8$ is as defined above.

Each group $R^3$ may contain a total of up to 550, preferably up to 400, more preferably up to 300, and especially up to 250 carbon atoms, with the provisos that up to a maximum of 3 $R^3$ groups can each simultaneously contain a total of greater than 40 carbon atoms and that the groups $R^3$ taken together contain a total of greater than 20 carbon atoms, e.g. up to 300, preferably up to 150, carbon atoms.

$R^3$ may be, for example, a $C_1$-$C_{40}$, preferably $C_1$-$C_{36}$, more preferably $C_1$-$C_{20}$, and especially $C_7$-$C_{18}$, alkyl group, or a polyalkenyl group derived from $C_2$-$C_6$ olefin monomers, preferably a polyisobutenyl group,

containing from 20 to 500 (corresponding to a number average molecular weight ($M_n$) range from 280 to 7000), preferably from 50 to 400 ($M_n$ range from 700 to 5600), and in particular from 50 to 200 ($M_n$ range from 700 to 2800), carbon atoms, any given $R^3$ group being optionally substituted with one or more, preferably one or two, substituent groups.

Examples of suitable substituent groups include halogen atoms (e.g. chlorine atoms), nitro, hydroxyl, cycloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl, alkylamido, aryl, alkaryl, heterocyclyl and polyoxyalkylene groups. When any of the foregoing substituents contain an alkyl or alkylene moiety, this may be linear or branched and may contain up to 12, preferably up to 6, and especially up to 4, carbon atoms A cycloalkyl group may contain from 3 to 8, preferably 3 to 6, carbon atoms. An aryl group may be any aromatic group, especially a phenyl or naphthyl group. An alkaryl group is an aryl group substituted by one or more, preferably one or two, alkyl groups. A heterocyclyl group may be any saturated or unsaturated ring system, e.g. a $C_5$-$C_7$ ring system, containing at least one heteroatom selected from oxygen, nitrogen and sulphur, 5- and 6- membered rings being especially preferred, e.g. a tetrahydrofuranyl, furanyl, piperidinyl, pyridinyl, tetrahydrothiophenyl or thiophenyl (thienyl) group.

$R^3$ may also be a group of formula II, IIIA or IIIB as defined above and is preferably a group of formula II or IIIB. Preferably in formula IIIB, D is derived from an unsaturated hydrocarbon being a polyalkene prepared from $C_2$-$C_6$ olefin monomers, e.g. polyisobutene, containing from 20 to 400, more preferably 35 to 300, and most preferably 50 to 200, carbon atoms.

x' in formula IV above is preferably 0, 1 or 2, most preferably 0 or 1.

It is preferred that in formula IV, $R^6$ and $R^{7'}$ together represent a group

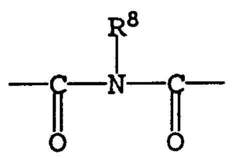

where $R^8$ is as defined above.

Particularly preferred compounds of formula I are those in which x is 1 and $R^3$ represents either a $C_{50}$-$C_{200}$ polyalkenyl group or a group of formula IIIB in which D is derived from an unsaturated hydrocarbon and x' in formula IV is 0; x is 2, one $R^3$ represents a $C_{50}$-$C_{200}$ polyalkenyl group and the other $R^3$ represents a group of formula II in which x' in formula IV is 1 and $R^{3'}$ represents a $C_{50}$-$C_{200}$ polyalkenyl group; or x is 3 to 4 and each $R^3$ independently represents a $C_7$-$C_{18}$ alkyl group.

It will be appreciated that the compounds of formula I are capable of existing as different geometric isomers. The present invention thus includes both the individual isomers and mixtures of such isomers.

The present invention also provides a process for the preparation of a compound of formula I as defined above which comprises reacting a compound of the general formula

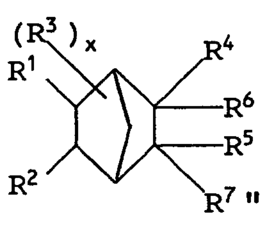

(VII)

wherein x, $R^1$ $R^2$, $R^3$, $R^4$, $R^5$ and $R^6$ are as defined above, provided that if a group $R^3$ represents a group of formula II, IIIA or IIIB then in formula IV $R^{7'}$ represents a group -CHO or -COOH or $R^6$ and $R^{7'}$ together represent a group

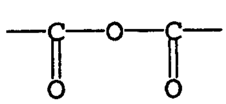

;

$R^{7''}$ represents a group -CHO or -COOH, or $R^6$ and $R^{7''}$ together represent a group

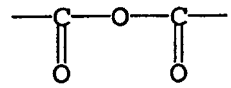 ;

with a compound of the general formula

B-H (VIII)

wherein, when $R^{7''}$ represents a -COOH group, then B represents a group X as defined above, or when $R^{7''}$ represents a -CHO group or when $R^6$ and $R^{7''}$ together represent a group

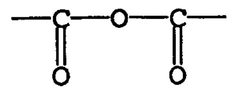 ;

then B represents a group $-NHR^8$ as defined above; and, where necessary or desired, selectively hydrogenating the product so obtained to reduce carbonyl (C=O) or imine (C=N) unsaturation.

The compounds of formulae VII and VIII are conveniently reacted in the presence of a solvent under reflux conditions. Suitable solvents include aromatic hydrocarbon solvents such as toluene and xylene, and chlorohydrocarbons such as dichloromethane.

Hydrogenation, if desired, of the reaction product of the compound of formula VII with the compound of formula VIII may be carried out in conventional manner, e.g as described in Advanced Organic Chemistry, Reactions, Mechanisms and Structure, by Jerry March, 3rd edition, published by Wiley-Interscience, pages 1093 to 1100.

The compounds of formulae VIII are known compounds or can be prepared by processes analogous to known processes.

Thus, for example, compounds of formula VIII where B is a group X wherein X represents a $C_{12}$-$C_{15}$ alkoxy group or a group of formula $R^{10}-O-(CH_2CH_2O)_p-$ where p and $R^{10}$ are as defined above are commercially available from member companies of the Royal Dutch/Shell Group under the "DOBANOL" trade mark.

Compounds of formula VIII where B is a group $-NHR^8$, e.g. 3-dimethylamino-1-propylamine, and compounds of formula V above in which A is -NH such as diethylene triamine, triethylene tetramine, tetraethylene pentamine and pentaethylene hexamine are commercially available from the Aldrich Chemical Company Ltd., (U.K).; and compounds of formula V above in which A is oxygen, as well as compounds of formula VI above are commercially available from the Texaco Chemical Company, Bellaire, Texas, U.S.A.

The compounds of formula VII are novel intermediates. Accordingly, the present invention further provides a compound of formula VII as defined above.

The compounds of formula VII may conveniently be prepared by a process which comprises reacting a compound of the general formula

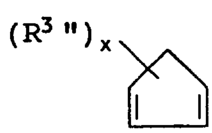

(IX)

in which x is as defined above and each $R^{3''}$ independently represents an optionally substituted alkyl or alkenyl group as defined for $R^3$ or a group of formula

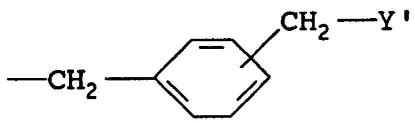

(II') ,

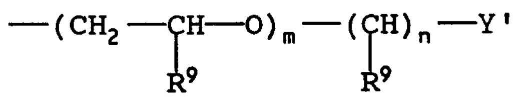

(IIIA')

or

—D—Y' (IIIB')

wherein m, n, $R^9$ and D are as defined above, and Y' represents a group of formula

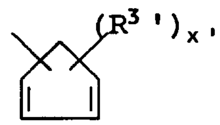

(X)

wherein x' and $R^{3'}$ are as defined above, each group $R^{3''}$ containing a total of up to 550 carbon atoms, with the provisos that up to a maximum of 3 $R^{3''}$ groups can each simultaneously contain a total of greater than 40 carbon atoms and that the groups $R^{3''}$ taken together contain a total of greater than 20 carbon atoms, with a compound of the general formula

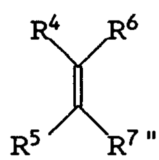

(XI)

wherein $R^4$, $R^5$, $R^6$ and $R^{7''}$ are as defined above; and, if desired, selectively hydrogenating the product obtained to reduce carbon-carbon double bond (C = C) unsaturation, e.g. as described in Advanced Organic Chemistry, Reactions, Mechanisms and Structure, by Jerry March, 3rd edition, published by Wiley-Interscience, pages 745 to 750.

The selective hydrogenation reaction to reduce C = C unsaturation is conveniently carried out in known manner, for example by using a palladium on charcoal catalyst.

The compounds of formula IX and XI are known compounds or can be prepared by processes analogous to known processes.

Thus, the compounds of formula IX may conveniently be prepared by reacting cyclopentadiene or dicyclopentadiene with at least one compound selected from the group consisting of

$R^{3'}$—L,

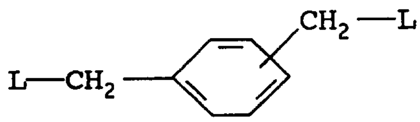

,

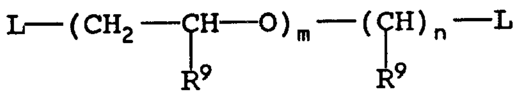

and

L—D—L

wherein m, n, $R^{3'}$, $R^9$ and D are as defined above and L represents a leaving group such as a halogen atom or a hydroxyl group, e.g. as described in U.S. Patents Nos. 4,721,823 and 5,012,023.

It will be appreciated that in the preparation of the compounds of formula IX using a reactant containing 2 leaving groups, L, it is possible for complex structures containing 3 or more cyclopentadiene rings linked to one another to be formed. These structures would be expected to react with compounds of formula XI in the same way as the compounds of formula IX, and the products so obtained would similarly be expected to react with compounds of formula VIII in the same way as the compounds of formula VII, to form analogues of the compounds of formula I. Indeed, it is the case that the formation of minor amounts of very high molecular weight materials has been observed in practice (as seen from gel permeation chromatography analysis). These high molecular weight materials would be expected to show the same good dispersancy properties as the compounds of formula I.

Particularly preferred compounds of formula XI are maleic anhydride and acrolein.

The compounds of formula I above may be used as dispersant additives in lubricating oils. Accordingly, the present invention further provides a lubricating oil composition comprising a major amount (more than 50%w) of a lubricating oil and a minor amount, preferably from 0.1 to 10%w, especially from 0.5 to 5%w, based on the total composition, of a compound of formula I as defined above.

Suitable lubricating oils are natural, mineral or synthetic lubricating oils.

Natural lubricating oils include animal and vegetable oils, such as castor oil. Mineral oils comprise the lubricating oil fractions derived from crude oils, coal or shale, which fractions may have been subjected to certain treatments such as clay-acid, solvent or hydrogenation treatments. Synthetic lubricating oils include synthetic polymers of hydrocarbons, modified alkylene oxide polymers, and ester lubricants, which are known in the art. These lubricating oils are preferably crankcase lubricating oils for spark-ignition and compression-ignition engines, but include also hydraulic lubricants, metal-working fluids and automatic transmission fluids.

Preferably the lubricating base oil component of the compositions according to the present invention is a mineral lubricating oil or a mixture of mineral lubricating oils, such as those sold by member companies of the Royal Dutch/Shell Group under the designations "HVI", or "XHVI" (trade mark).

The viscosity of the lubricating base oils present in the compositions according to the present invention may vary within wide ranges, and is generally from 3 to 35 $mm^2/s$ at 100 ° C.

The lubricating oil compositions according to the present invention may contain various other additives, known in the art, such as viscosity index improvers, e.g. linear or star-shaped polymers of a diene such as isoprene or butadiene, or a copolymer of such a diene with optionally substituted styrene. These copolymers are suitably block copolymers and are preferably hydrogenated to such an extent as to saturate most of the olefinic unsaturation. Other suitable additives include dispersant V.I. improvers such as those based on block copolymers, or polymethacrylates, extreme pressure/anti-wear additives such as zinc or sodium dithiophosphates, ashless dispersants such as polyolefin-substituted succinimides, e.g. those described in GB-A-2 231 873, anti-oxidants, friction modifiers or metal-containing detergents such as phenates, sulphonates, alkylsalicylates or naphthenates, all of which detergents may be overbased.

The compounds of formula I above may also be used as dispersant additives in fuels. Accordingly, the present invention further provides a fuel composition comprising a major amount (more than 50w) of a fuel and a minor amount, preferably from 0.01 to 2%w, especially from 0.01 to 0.5%w, based on the total composition, of a compound of formula I as defined above.

Suitable fuels include hydrocarbon base fuels boiling essentially in the gasoline boiling range from 30 to 230 °C. These base fuels may comprise mixtures of saturated, olefinic and aromatic hydrocarbons. They can be derived from straight-run gasoline, synthetically produced aromatic hydrocarbon mixtures, thermally or catalytically cracked hydrocarbon feedstocks, hydrocracked petroleum fractions or catalytically reformed hydrocarbons.

The fuel compositions according to the present invention may contain various other additives known in the art such as a lead compound as anti-knock additive; antioxidants such as phenolics, e.g., 2,6-di-tert-butylphenol or phenylenediamines, e.g., N,N'-di-sec-butyl-p-phenylenediamine; dyes; metal deactivators; dehazers such as polyester-type ethoxylated alkylphenolformaldehyde resins; corrosion inhibitors, such as a polyhydric alcohol ester of a succinic acid derivative having on at least one of its alpha-carbon atoms an unsubstituted or substituted aliphatic hydrocarbon group having 20 to 500 carbon atoms, for example, pentaerythritol diester of polyisobutylene-substituted succinic acid, the polyisobutylene group having a number average molecular weight of 950; antiknock additives other than lead compounds such as methyl cyclopentadienyl-manganese tricarbonyl or ortho-azidophenol; co-antiknock additives such as benzoylacetone; or carrier fluids such as a polyether e.g. a $C_{12}$-$C_{15}$ alkyl-substituted propylene glycol ("SAP 949" which is commercially available from member companies of the Royal Dutch/Shell group), "HVI" or "XHVI" base oil, or a polyolefin derived from $C_2$-$C_6$ monomers, e.g. polyisobutylene having from 20 to 175, particularly 35 to 150, carbon atoms.

The lubricating oil and fuel compositions of the invention may be prepared by adding one or more compounds of formula I above separately to a lubricating oil or fuel. Conveniently, an additive concentrate is blended with the lubricating oil or fuel. Such a concentrate generally comprises an inert carrier fluid and one or more additives in a concentrated form. Hence the present invention further provides an additive concentrate comprising an inert carrier fluid and from 10 to 80%w, based on the total concentrate, of a compound of formula I as defined above.

Suitable inert carrier fluids are hydrocarbons and mixtures of hydrocarbons with alcohols or ethers, such as methanol, ethanol, propanol, 2-butoxyethanol or methyl tert-butyl ether. For example, the carrier fluid may be an aromatic hydrocarbon solvent such as toluene, xylene, mixtures thereof or mixtures of toluene or xylene with an alcohol. Alternatively, the carrier fluid may be a mineral base oil, such as those sold by member companies of the Royal Dutch/Shell Group under the designations "HVI" or "XHVI" (trade mark) e.g. "HVI 60" base oil.

The present invention still further provides the use of a compound of formula I as defined above as a dispersant additive.

The invention will be further understood from the following illustrative examples.

## Example 1

(i) Preparation of a multiply alkylated cyclopentadiene (alcohol method)

A mixture of $C_9$-$C_{11}$ alcohols (424 g, 2.65 mol) having mean relative molecular mass 160 ± 3 as determined by gas chromatography (available under the trade mark "Dobanol 91" from member companies of the Royal Dutch/Shell Group) was deoxygenated and then heated together with powdered potassium hydroxide (87%) (10.3 g, 0.16 mol) and dicyclopentadiene (6.6 g, 0.05 mol) to 180 °C in a reaction vessel equipped with a Dean and Stark trap. As soon as water began to collect in the Dean and Stark trap, further dicyclopentadiene (28.4 g, 0.215 mol) was added portionwise to the reaction mixture over a period of 4 hours. The temperature of the reaction mixture rose gradually to approximately 240 °C and was held at that temperature until no further water was produced. Once cooled, the reaction mixture was diluted with hexane (500 ml), washed with water (8 x 500 ml) until a neutral pH was obtained, and then dried over magnesium sulphate. The hexane was subsequently removed under reduced pressure to give a crude product (430 g). Purification of the crude product to remove unreacted alcohol using a wiped film evaporator yielded 241 g of the desired product, a multiply alkylated cyclopentadiene containing, on average, 3.4 $C_9$-$C_{11}$-alkyl substituent groups as later determined by $^{13}C$ Nuclear Magnetic Resonance Spectroscopy (NMR) following step (ii) below.

(ii) Preparation of a succinic anhydride derivative of a multiply alkylated cyclopentadiene

The multiply alkylated cyclopentadiene obtained in (i) above (11.4 g, 20 mmol) and maleic anhydride (3 g, 30 mmol) were dissolved in toluene (200 ml) and heated at reflux under a nitrogen blanket for 2 hours. The reaction mixture was allowed to cool and the toluene was subsequently removed under reduced pressure to leave a residue. Purification of the residue by flash chromatography on silica using a petroleum spirit/diethyl ether mixture (95:5 v/v) as eluant gave the desired product, the succinic anhydride derivative of the multiply alkylated cyclopentadiene, as a pale yellow oil (7.4 g). Infrared

spectral analysis of the product showed a peak ($v_{max}$) at 1855 $cm^{-1}$ (m) and at 1778 $cm^{-1}$ (vs).

(iii) Preparation of a bis imide adduct, a compound of formula I in which $R^1$ and $R^2$ together represent a carbon-carbon single bond, x is 3.4, each $R^3$ is a $C_9$-$C_{11}$ alkyl, $R^4$ and $R^5$ are each hydrogen, $R^6$ and $R^7$ together represent

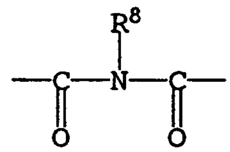

and two groups $R^8$ together form a bridging group $-(CH_2)_2-NH-(CH_2)_2-NH-(CH_2)_2-$, **i.e.**

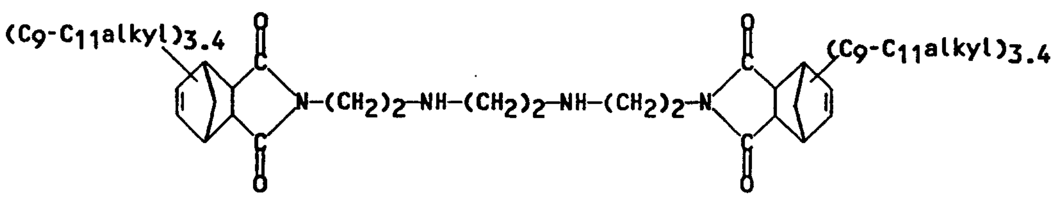

A solution of a succinic anhydride derivative prepared as described in (ii) above (15 g, 0.025 mol) and triethylene tetramine (1.83 g, 0.0125 mol) in toluene (200 ml) was refluxed for 2 hours, with removal of water using a Dean and Stark trap. Toluene was subsequently removed under reduced pressure and the remaining residue was purified by flash chromatography on silica using a dichloromethane/methanol mixture (95:5 v/v) as eluant to give the desired product, a bis imide, as a pale yellow oil (15 g). Infrared spectral analysis of the product showed $v_{max}$ at 1765 $cm^{-1}$ (m) and 1698 $cm^{-1}$ (s).

Example 2

(i) Preparation of a hydrogenated succinic anhydride derivative of a multiply alkylated cyclopentadiene

A solution of a succinic anhydride derivative prepared as in Example 1 (ii) (4 g, 40 mmol) in heptane (250 ml) was introduced into an autoclave together with 5% palladium on charcoal (1 g) as hydrogenation catalyst. The autoclave was pressurised with hydrogen (6 MPa) and the contents of the autoclave stirred for 2 days. The autoclave contents were subsequently filtered through "Hyflo" (trade mark) filter aid to remove the catalyst and then heptane was removed under reduced pressure from the resulting filtrate to yield the product as a yellow oil (4 g) (15% starting material, 85% hydrogenated succinic anhydride derivative as determined by $^{13}C$ NMR).

(ii) Preparation of a bis imide adduct, a compound of formula I in which $R^1$ and $R^2$ are each hydrogen, x is 3.4, each $R^3$ is a $C_9$-$C_{11}$ alkyl, $R^4$ and $R^5$ are each hydrogen, $R^6$ and $R^7$ together represent

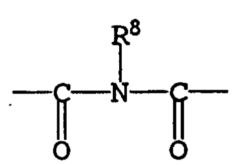

and two groups $R^8$ together form a bridging group $-(CH_2)_2-NH-(CH_2)_2-NH-(CH_2)_2-$, **i.e.**

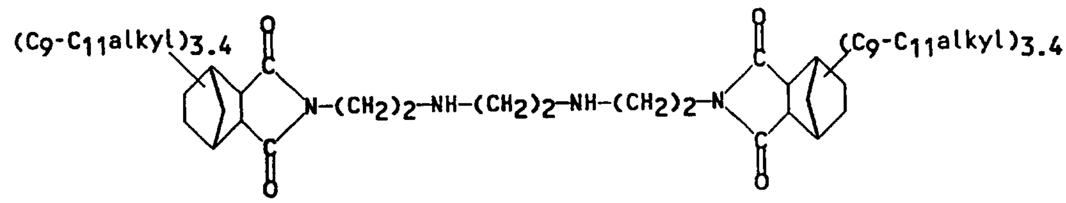

A solution of a product prepared as described in (i) above (12 g, 20 mmol) and triethylene tetramine (1.4 g, 9.5 mmol) in toluene (300 ml) was refluxed for 2 hours, with removal of water using a Dean and Stark trap. Toluene was subsequently removed under reduced pressure and the remaining residue was purified by flash chromatography on silica using a dichloromethane/methanol mixture (95:5 v/v) as eluant to give the desired product, a bis imide, as a pale yellow oil (7.2 g). Infrared spectral analysis of the product showed $v_{max}$ at 1768 $cm^{-1}$ (m) and 1702 $cm^{-1}$ (s).

Example 3

(i) Preparation of a multiply alkylated cyclopentadiene (phase transfer method)

1-Bromooctadecane (500 g, 1.5 mol), cyclopentadiene (20 g, 0.3 mol) and an aqueous solution of potassium hydroxide (50%) (400 ml, 6.0 mol) were heated, in the presence of [methyltrialkyl($C_8$-$C_{10}$)-ammonium chloride] (a phase transfer catalyst available commercially under the trade mark "Adogen 464"), at 70 ° C for half an hour and then at 90 ° C for a further 2 hours. The reaction mixture was cooled, diluted with toluene (400 ml), then washed with water (500 ml) and filtered through "Celite" filter agent. The filtrate was dried over magnesium sulphate and toluene was removed under reduced pressure to yield 440g of the desired product, an alkylated cyclopentadiene containing, on average, 3.8 $C_{18}$-alkyl substituent groups as later determined by $^{13}C$ NMR following step (ii) below.

(ii) Preparation of a succinic anhydride derivative of a multiply alkylated cyclopentadiene

The multiply alkylated cyclopentadiene obtained in (i) above (100 g, 0.1 mol) and maleic anhydride (11 g, 0.11 mol) were dissolved in toluene (300 ml) and heated at reflux under a nitrogen blanket for 1 hour. The reacton mixture was allowed to cool and the toluene was subsequently removed under reduced pressure to leave a residue. Purification of the residue by flash chromatography on silica using a petroleum spirit/diethyl ether mixture (90:10 v/v) as eluant gave the desired product, the succinic anhydride derivative of the multiply alkylated cyclopentadiene, as a pale yellow solid (50 g). Infrared spectral analysis of the product showed $v_{max}$ at 1850 $cm^{-1}$(m) and 1780 $cm^{-1}$ (s).

(iii) Preparation of a mono imide adduct, a compound of formula I in which $R^1$ and $R^2$ together represent a carbon-carbon single bond, x is 3.8, each $R^3$ is a $C_{18}$ alkyl, $R^4$ and $R^5$ are each hydrogen, $R^6$ and $R^7$ together represent

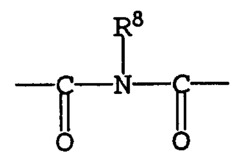

and $R^8$ is $-(CH_2)_2-NH-(CH_2)_2-NH-(CH_2)_2-NH_2$, **i.e.**

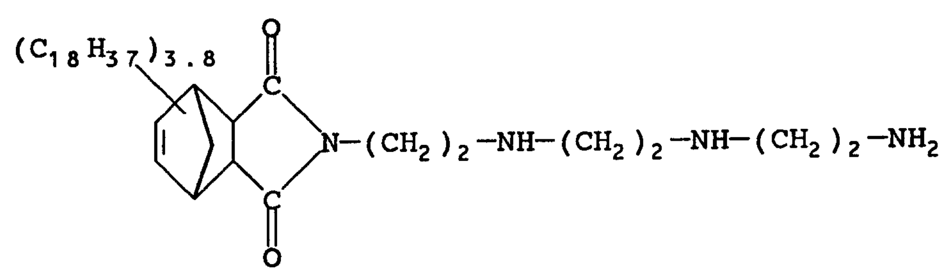

A solution of the succinic anhydride derivative obtained in (ii) above (15 g, 15 mmol) and triethylene tetramine (22 g, 0.15 mol) in xylene (300 ml) was refluxed for 2 hours with removal of water using a Dean and Stark trap. Xylene was subsequently removed under reduced pressure and the remaining residue was dissolved in petroleum spirit (300 ml) and washed with a methanol/water mixture (90:10 v/v) (2 x 100 ml). The solution was then dried over magnesium sulphate, filtered, and the petroleum spirit was removed under reduced pressure to yield the desired product, a mono imide, as a pale yellow oil (15.4 g). Infrared spectral analysis of the product showed $v_{max}$ at 1770 $cm^{-1}$(m) and 1700 $cm^{-1}$ (s).

Examples 4 to 26

By processes similar to those described in Examples 1 to 3 above, further compounds according to the invention were prepared as detailed in Tables I and II below in which the following abbreviations are used:-

DAP : 3-dimethylamino-1-propylamine
DETA : diethylene triamine
TETA : triethylene tetramine
TEPA : tetraethylene pentamine
PEHA : pentaethylene hexamine
D400 : "Jeffamine D400" polyoxyalkylene amine, a compound of formula V above in which A is -O-, r is 1, each $R^{11}$ represents a methyl group and s is 4 to 5
D2000: "Jeffamine D2000" polyoxyalkylene amine, a compound of formula V above in which A is -O-, r is 1, each $R^{11}$ represents a methyl group and s is 33

Table I

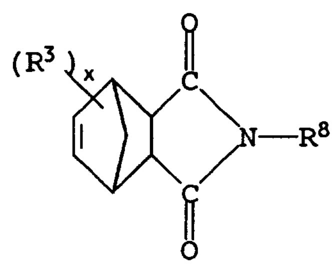

| Example No. | x | $R^3$ | Amine from which $N-R^8$ is derived |
|---|---|---|---|
| 4 | 4 | $C_{10}$-alkyl | DAP |
| 5 | 4 | $C_{10}$-alkyl | TETA |
| 6 | 3.6 | $C_{10}$-alkyl | D400 |
| 7 | 3.6 | $C_{10}$-alkyl | D2000 |
| 8 | 3.8 | $C_{18}$-alkyl | DAP |
| 9 | 3.6 | $C_7$-$C_9$-alkyl | DAP |
| 10 | 3.6 | $C_7$-$C_9$-alkyl | DAP |

Table II

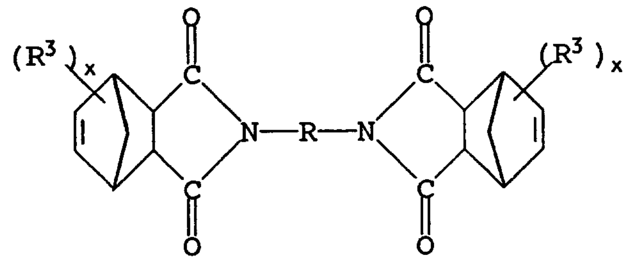

| Example No. | x | $R^3$ | Amine from which N-R-N is derived |
|---|---|---|---|
| 11 | 3.4 | $C_9$-$C_{11}$-alkyl | TEPA |
| 12 | 3.6 | $C_{10}$-alkyl | D400 |
| 13 | 3.6 | $C_{10}$-alkyl | D2000 |
| 14 | 3.8 | $C_{18}$-alkyl | TETA |
| 15 | 4 | $C_9$-$C_{11}$-alkyl | DETA |
| 16 | 4 | $C_9$-$C_{11}$-alkyl | TETA |
| 17 | 4 | $C_9$-$C_{11}$-alkyl | TEPA |
| 18 | 4 | $C_9$-$C_{11}$-alkyl | PEHA |
| 19 | 3.6 | $C_7$-$C_9$-alkyl | DETA |
| 20 | 3.6 | $C_7$-$C_9$-alkyl | TETA |
| 21 | 3.6 | $C_7$-$C_9$-alkyl | TEPA |
| 22 | 3.6 | $C_7$-$C_9$-alkyl | PEHA |
| 23 | 3.7 | $C_9$-$C_{11}$-alkyl | TETA |
| 24 | 3.7 | $C_{16}$-$C_{18}$-alkyl | TETA |
| 25 | 3.7 | $C_{16}$-$C_{18}$-alkyl | TEPA |
| 26 | 3.7 | $C_{16}$-$C_{18}$-alkyl | PEHA |

Example 27

(i) Preparation of an acrolein derivative of a multiply alkylated cyclopentadiene

Acrolein (10 g, 0.14 mol) was added to a stirred solution of the multiply alkylated cyclopentadiene

containing an average of 3.4 $C_9$-$C_{11}$-alkyl substituent groups obtained in Example 1(i) above (84 g, 0.14 mol) in toluene (300 ml). The reaction mixture was refluxed for 1 hour, cooled and the toluene was subsequently removed under reduced pressure to leave a residue. Purification of the residue by flash chromatography on silica using a petroleum spirit/diethyl ether mixture (90:10 v/v) as eluant gave the desired product, the acrolein derivative of the multiply alkylated cyclopentadiene, as a pale yellow oil (15 g). Infrared spectral analysis of the product showed $v_{max}$ at 1718 $cm^{-1}$ (s).

(ii) Preparation of a bis imine adduct

Triethylene tetramine (2.4 g, 0.166 mol) was added to an acrolein derivative prepared as described in (i) above (20 g, 0.033 mol) and magnesium sulphate in dry dichloromethane (100 ml). The reaction mixture was stirred for 1 hour at ambient temperature (20 °C) and the progress of the reaction was monitored by infrared spectral analysis. The magnesium sulphate was then filtered off and the dichloromethane was removed under reduced pressure to leave a crude product containing the desired bis imine adduct.

(iii) Hydrogenation of bis imine adduct, to form a compound of formula I in which $R^1$ and $R^2$ together represent a carbon-carbon single bond, x is 3.4, each $R^3$ is a $C_9$-$C_{11}$ alkyl, $R^4$, $R^5$ and $R^6$ are each hydrogen, $R^7$ is $-CH_2-NHR^8$ and two groups $R^8$ together form a bridging group $-(CH_2)_2-NH-(CH_2)_2-NH-(CH_2)_2-$, **i.e.**

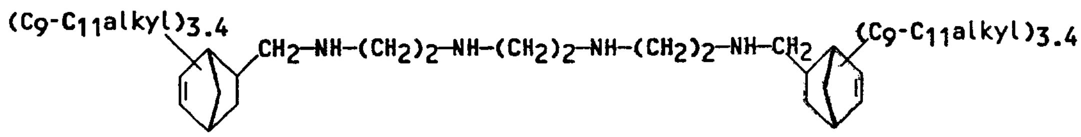

The crude product obtained in (ii) above was dissolved in dry tetrahydrofuran and the resulting solution was cooled to -30 °C. Lithium aluminium hydride (1.6 g, 0.04 mol) was added to the cooled solution to selectively hydrogenate the imine (C = N) bonds in the bis imine adduct and the reaction mixture was stirred at room temperature for 2 hours. The reaction mixture was then cooled to 0 °C, to which water was added slowly. Filtration of the reaction mixture to remove the solids followed by removal of the tetrahydrofuran under reduced pressure yielded a residue. Purification of the residue by flash chromatography on silica using a methanol/dichloromethane mixture (10:90 v/v) as eluant gave the desired product, a bis amine compound, as a yellow oil (15 g).

Example 28

(i) Preparation of a mono-polyisobutylene-substituted cyclopentadiene

Monochlorinated polyisobutylene (PIB) ($M_n$ 950) (50 g, 0.05 mol), cyclopentadiene (13 g, 0.2 mol), a 50% aqueous solution of potassium hydroxide (54 g), and "Adogen 464" phase transfer catalyst (1 g) in toluene (50 ml) were introduced into a reaction vessel and stirred for 10 minutes at 5 °C. The reaction mixture was heated for 2 hours at 80 °C, then cooled to ambient temperature (20 °C) and diluted with petroleum spirit (600 ml). The solution was washed with water (5 x 200ml), dried over magnesium sulphate and concentrated under reduced pressure to give the crude, mono-PIB-substituted cyclopentadiene (52 g).

(ii) Preparation of a succinic anhydride derivate of a mono-PIB-substituted cyclopentadiene

The crude product obtained in (i) above (52 g) was dissolved in toluene (200 ml), to which maleic anhydride (10 g, 0.1 mol) was added. The reaction mixture was then heated at reflux under a nitrogen blanket for 1 hour. When cooled, the reaction mixture was concentrated under reduced pressure and the residue thus obtained was purified by flash chromatography on silica using, as eluant, a dichloromethane/petroleum spirit mixture (50:50 v/v) to yield the desired product, the succinic anhyrdride derivative of the mono-PIB-substituted cyclopentadiene, as a pale yellow oil (14.5 g).

(iii) Preparation of a bis imide adduct, a compound of formula I in which $R^1$ and $R^2$ together represent a carbon-carbon single bond, x is 1, $R^3$ is a polyisobutenyl chain, $R^4$ and $R^5$ are each hydrogen, $R^6$ and $R^7$ together represent

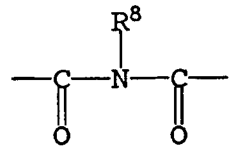

and two groups $R^8$ together form a bridging group $-(CH_2)_2-NH-(CH_2)_2-NH-(CH_2)_2-$, **i.e.**

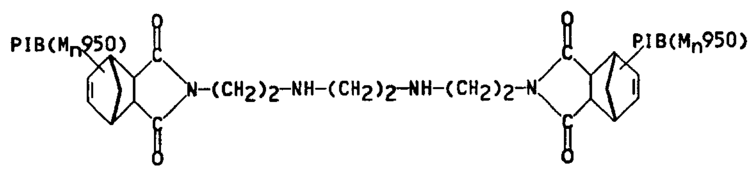

A solution of a succinic anyhydride derivative prepared as described in (ii) above (27 g, 0.0022 mol) and triethylene tetramine (1.6 g, 0.0011 mol) in toluene (200 ml) was refluxed for 1 hour, with removal of water using a Dean and Stark trap. Toluene was subsequently removed under reduced pressure and the remaining residue was purified by flash chromatography on silica using a dichloromethane/methanol mixture (95:5 v/v) as eluant to give the desired product, a bis imide, as a yellow oil (28 g).

Example 29

In this example, essentially the same procedure as described in Example 28 was followed except that in step (i) a PIB of $M_n$ 1800 was used and in step (iii) pentaethylene hexamine (0.45 g, 0.00195 mol) was used. A bis imide product was obtained as a brown viscous liquid (8.9 g), i.e. a compound of formula I in which $R^1$ and $R^2$ together represent a carbon-carbon single bond, x is 1, $R^3$ is a polyisobutenyl chain, $R^4$ and $R^5$ are each hydrogen, $R^6$ and $R^7$ together represent

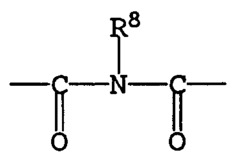

and two groups $R^8$ together form a bridging group $-(CH_2)_2-[NH-(CH_2)_2-]_4$, **i.e.**

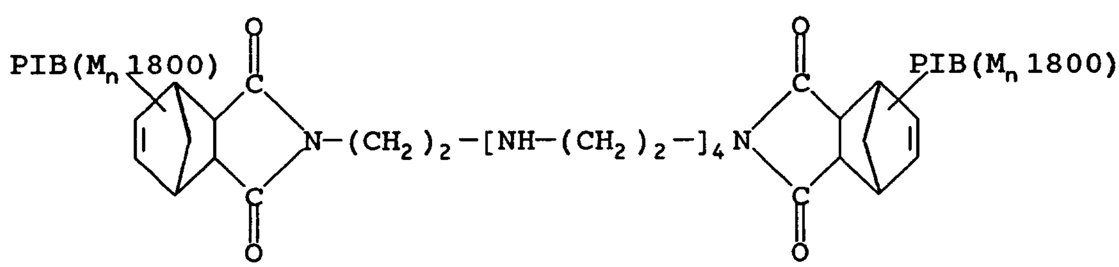

Example 30

(i) Preparation of a bridged alkylated cyclopentadiene

An ice cold, 50% aqueous solution of potassium hydroxide (100 g) was added, with stirring, to an ice cold mixture of cyclopentadiene (14 g, 0.22 mol), monochlorinated polyisobutylene (PIB) ($M_n$ 1378) (200 g, 0.2 mol),$\alpha$,$\alpha$'-dichloroxylene (17.5 g, 0.1 mol) and "Adogen 464" phase transfer catalyst (5 g) in toluene (400 ml). The reaction mixture was stirred at ambient temperature (20 °C) for 15 minutes and then at 80 °C for 2 hours. Once cooled, the reaction mixture was diluted with petroleum spirit (800 ml),

washed with water (5 x 300 ml) until a neutral pH was obtained, and then dried over magnesium sulphate. Concentration under reduced pressure yielded the crude, xylyl-bridged, mono-PIB-substituted cyclopentadiene (220 g).

(ii) Preparation of a sucinic anhydride derivative of a bridged alkylated cyclopentadiene

The crude product obtained in (i) above (220 g, 0.2 mol) and maleic anhydride (19 g, 0.2 mol) were dissolved in toluene (800 ml) and heated at reflux under a nitrogen blanket for 1 hour. The reaction mixture was allowed to cool and the toluene was subsequently removed under reduced pressure to leave a residue. Purification of the residue by flash chromatography on silica using, as eluant, petroleum spirit and then dichloromethane, gave the desired product, the succinic anhydride derivative of the xylyl-bridged, mono-PIB-substituted cyclopentadiene, as a brown oil (220 g). Infrared spectral analysis of the product showed $v_{max}$ at 1861 $cm^{-1}$(m) and 1783 $cm^{-1}$ (vs).

(iii) Preparation of an imide, a compound of formula I in which $R^1$ and $R^2$ together represent a carbon-carbon single bond, $R^4$ and $R^5$ are each hydrogen, $R^6$ and $R^7$ together represent

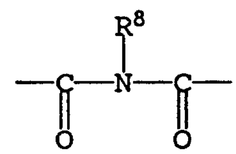

$R^8$ is $-(CH_2)_2-[NH-(CH_2)_2-]_3-NH_2$, x is 2, one $R^3$ is a polyisobutenyl chain and the other $R^3$ is a group of formula II above in which in formula IV x' is 1, $R^{3'}$ is a polyisobutenyl chain, $R^6$ and $R^{7'}$ together represent

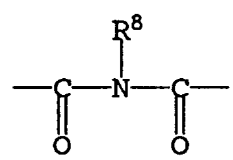

and $R^1$, $R^2$, $R^4$, $R^5$ and $R^8$ are as defined above, **i.e.**

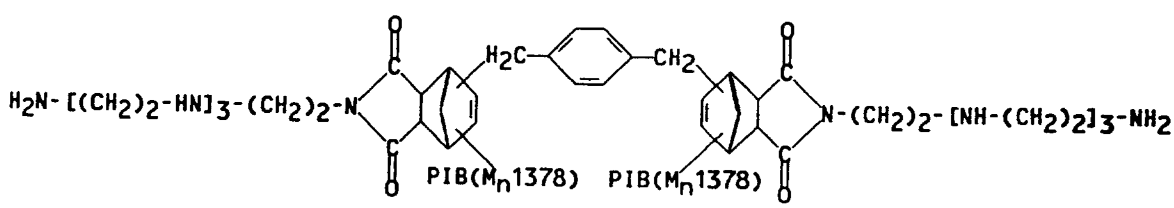

A solution of the succinic anhydride derivative obtained in (ii) above (20 g, 0.0143 mol) and tetraethylene pentamine (1.35 g, 0.009 mol) in toluene (300 ml) was refluxed for 1.5 hours with removal of water using a Dean and Stark trap. Toluene was subsequently removed under reduced pressed and the remaining residue was purified by flash chromatography on silica using, as eluant, dichloromethane and then a dichloromethane/methanol mixture (90:10 v/v) to give the desired product, an imide, as a brown oil (21 g). Infrared spectral analysis of the product showed $v_{max}$ at 1770 $cm^{-1}$ (m) and 1702 $cm^{-1}$ (s).

Examples 31 to 39

The procedure of Example 30 was repeated with the variations shown in Table III below to produce further compounds of formula I. In Table III, the following abbreviations are used:

CPD : cyclopentadiene
DCX : α,α'-dichloroxylene
PIB : polyisobutylene
MALA : maleic anhydride
TETA : triethylene tetramine

TEPA : tetraethylene pentamine
PEHA : pentaethylene hexamine
HEPA : higher ethylene polyamines (essentially a mixture of tetraethylene pentamine, pentaethylene hexamine and hexaethylene heptamine in a molar ratio of 1:2:1)

Table III

| Example No. | PIB $M_n$ | Molar ratio of reactants (CPD : DCX : MALA) | Amine |
|---|---|---|---|
| 31 | 950 | 1 : 0.25 : 1 | PEHA |
| 32 | 950 | 1 : 0.13 : 1 | TETA |
| 33 | 950 | 1 : 0.17 : 1 | TETA |
| 34 | 950 | 1 : 0.5 : 1 | TETA |
| 35 | 1800 | 1 : 0.5 : 1 | TEPA |
| 36 | 2400 | 1 : 1 : 1 | PEHA |
| 37 | 2400 | 1 : 1 : 1 | HEPA |
| 38 | 2400 | 1 : 2 : 1 | PEHA |
| 39 | 2400 | 1 : 2 : 1 | HEPA |

Example 40

(i) Preparation of a bridged cyclopentadiene

An ice cold, 50% aqueous solution of potassium hydroxide (200 ml) was added, with stirring, to an ice cold mixture of cyclopentadiene (20 g, 0.3 mol), dichlorinated polyisobutylene (PIB) ($M_n$ 1800) (138 g, 0.076 mol) and "Adogen 464" phase transfer catalyst (1 g) in toluene (250 ml). The reaction mixture was stirred at 5 °C for 10 minutes and then at 80 °C for 2 hours. Once cooled, the reaction mixture was diluted with petroleum spirit (500 ml), washed with water (5 x 200 ml) until a netural pH was obtained, and then dried over magnesium sulphate. Concentration under reduced pressure yielded the crude, PIB-bridged cyclopentadiene (100g).

(ii) Preparation of a succinic anhydride derivative of a bridged cyclopentadiene

The crude product obtained in (i) above (100 g) and maleic anhydride (10 g, 0.1 mol) were dissolved in toluene (200 ml) and heated at reflux under a nitrogen blanket for 1 hour. The reaction mixture was allowed to cool and the toluene was subsequently removed under reduced pressure to leave a residue. The residue was purified by dissolving in petroleum spirit (1000 ml) and spinning the solution in a centrifuge at 2500 rpm for 30 minutes. The supernatant liquid was then drawn off and concentrated under reduced pressure to give the desired product, the succinic anhydride derivative of the PIB-bridged cyclopentadiene, as a clear brown oil (100 g). Infrared spectral analysis of the product showed $v_{max}$ at 1783 $cm^{-1}$ (s) and 1853 $cm^{-1}$ (m).

(iii) Preparation of an imide, a compound of formula I in which $R^1$ and $R^2$ together represent a carbon-carbon single bond, $R^4$ and $R^5$ are each hydrogen, $R^6$ and $R^7$ together represent

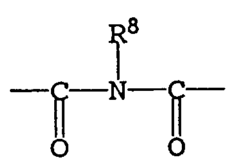

$R^8$ is $-(CH_2)_2-[NH-(CH_2)_2-]_3-NH_2$, x is 1, and $R^3$ is a group of formula IIIB above in which D is derived from a polyisobutylene ($M_n$ 1800), and in formula IV x' is 0, $R^6$ and $R^7$ together represent

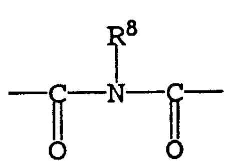

and $R^1$, $R^2$, $R^4$, $R^5$ and $R^8$ are as defined above, **i.e.**

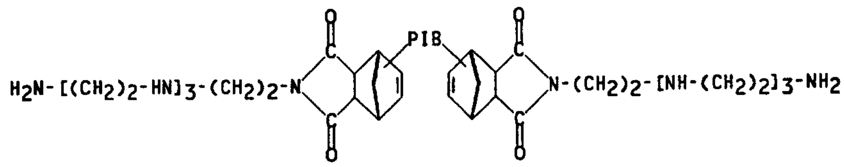

A solution of the succinic anhydride derivative obtained in (ii) above (20 g, 0.0143 mol) and tetraethylene pentamine (1.35 g, 0.009 mol) in toluene (250 ml) was refluxed for 2 hours with removal of water using a Dean and Stark trap. Toluene was subsequently removed under reduced pressure and the remaining residue was purified by flash chromatography on silica using, as eluant, dichloromethane and then a dichloromethane/methanol mixture (90:10 v/v) to give the desired product, an imide, as a brown oil (21 g). Infrared spectral analysis of the product showed $v_{max}$ at 1770 $cm^{-1}$ (m) and 1702 $cm^{-1}$ (vs).

Example 41

(i) Carbon Black Dispersancy Test (CBDT) (British Rail Publication BR 669 : 1984)

Samples of a SAE 15W40 Middle East lubricating oil containing a commercial package of a zinc dialkyldithiophosphate, an overbased calcium alkyl salicylate and VI improver, were modified by incorporation of the imide products of Examples 1, 2, 7, 13, 14, 24 to 26, 38 and 39 to give oils containing the products at a concentration of 1%w active matter. 3%w of carbon black was then added to each oil and (percentage) increase in kinematic viscosity at 60°C was determined, using an Ubbelohde viscometer. A low result indicates good performance. The absolute values obtained are dependent on the active surface area of the carbon black used, and therefore comparative series should be tested with identical samples of carbon black. The tests were carried out using either "Flamruss" (trade mark) or "Cabot Carbon Elftex 460" (trade mark) as the carbon black.

(ii) Fluoroelastomer Seal Compatibility Test (FSCT)

The imide products of Examples 1, 2, 7, 13, 14, 24 to 26, 38 and 39 were incorporated in lubricating oils to give concentrations of 1.5%w active matter and tested for compatibility with fluoroelastomer seal materials according to the method of DIN 53504 and, specifically, Daimler Benz specification DB 6615. Percentage reduction in tensile strength (TS) and elongation at break (EB) were assessed. The test results depend upon the particular seal materials used, and therefore comparative series should be tested with seals from consistent batches. A low result indicates good performance.

Results of these tests are given in Table IV following:

Table IV

| Imide Product of Example | CBDT (%) | | FSCT | |
|---|---|---|---|---|
| | a | b | TS (%) | EB (%) |
| 1 | - | 202 | 36 | 33 |
| 2 | - | 174 | 27 | 25 |
| "SAP 220" | - | 195 | 31 | 29 |
| 7 | 48 | - | 6 | 6 |
| 13 | 43 | - | 4 | 3 |
| "SAP 220" | 32 | - | 24 | 23 |
| 14 | 46 | - | 29 | 25 |
| "SAP 220" | 32 | - | 34 | 29 |
| 24 | 50 | - | 17 | 22 |
| 25 | 44 | - | 16 | 18 |
| 26 | 40 | - | 22 | 21 |
| "SAP 220" | 46 | - | 15 | 16 |
| 38 | 27 | - | 23 | 27 |
| 39 | 19 | - | 13 | 17 |
| "SAP 220" | 43 | - | 23 | 21 |

a denotes "Flamruss" carbon black

b denotes "Cabot Carbon Elftex 460" carbon black "SAP 220" is a commercially available polyisobutylene-derived bis-succinimide ashless dispersant (polyisobutylene of $M_n$ 950, amine is TETA)

## Claims

1. A compound of the general formula

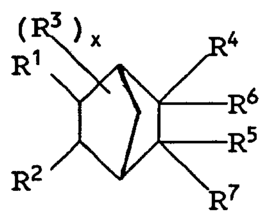

(I)

in which $R^1$ and $R^2$ each represent a hydrogen atom, or together represent a carbon-carbon single bond; each of $R^4$ and $R^5$ independently represents a hydrogen atom, or a $C_1$-$C_{20}$ alkyl or phenyl group, each of which may be optionally substituted; $R^6$ represents a hydrogen atom, or a $C_1$-$C_{20}$ alkyl or phenyl group, each of which may be optionally substituted and $R^7$ represents a group $-CH_2-NHR^8$ in which $R^8$ represents an optionally substituted alkyl group, or a group -COX wherein X represents an optionally substituted alkoxy group or $-NHR^8$ where $R^8$ is as defined above; or $R^6$ and $R^7$ together represent a group

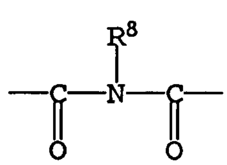 , 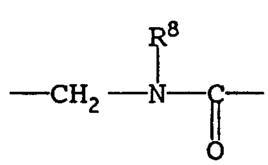 , or 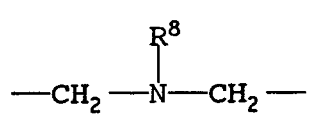

where $R^8$ is as defined above; x is 1 to 6; and each $R^3$ independently represents an optionally substituted alkyl or alkenyl group or a group of formula

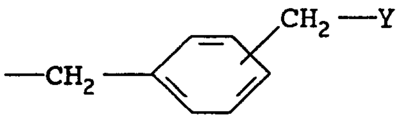

(II) ,

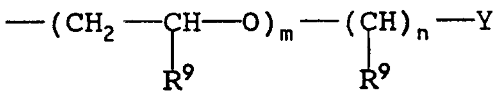

(IIIA)

or

—D—Y (IIIB)

wherein m is in the range 0 to 25, n is in the range 1 to 20, each $R^9$ independently represents a hydrogen atom or a methyl or ethyl group, D is derived from a saturated or unsaturated hydrocarbon containing from 20 to 500 carbon atoms, and Y represents a group of formula

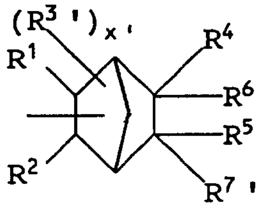

(IV)

in which $R^1$, $R^2$, $R^4$, $R^5$ and $R^6$ are as defined above; x' is 0 to 5; $R^{3'}$ is as defined for $R^3$ other than a group of formula II, IIIA, or IIIB, $R^{7'}$ represents a group $R^7$ as defined above or a group -CHO or -COOH, or $R^6$ and $R^{7'}$ together represent a group

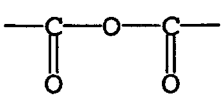 , 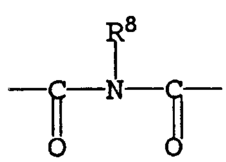 ,

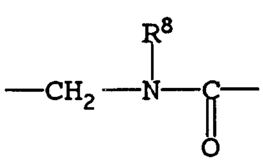 or 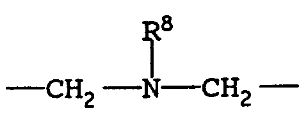

where $R^8$ is as defined above, each group $R^3$ containing a total of up to 550 carbon atoms, with the provisos that up to a maximum of 3 $R^3$ groups can each simultaneously contain a total of greater than 40 carbon atoms and that the groups $R^3$ taken together contain a total of greater than 20 carbon atoms.

**2.** A compound according to claim 1, wherein $R^1$ and $R^2$ together represent a carbon-carbon single bond.

**3.** A compound according to claim 1 or 2, wherein each $R^3$ independently represents a $C_1$ -$C_{40}$ alkyl group, a $C_{50}$-$C_{200}$ polyalkenyl group, or a group of formula II or IIIB as defined in claim 1.

**4.** A compound according to claim 3, wherein x is 1 and $R^3$ represents either a $C_{50}$-$C_{200}$ polyalkenyl group or a group of formula IIIB in which D is derived from an unsaturated hydrocarbon and x' in formula IV is 0; x is 2, one $R^3$ represents a $C_{50}$-$C_{200}$ polyalkenyl group and the other $R^3$ represents a group of formula II in which x' in formula IV is 1 and $R^{3'}$ represents a $C_{50}$-$C_{200}$ polyalkenyl group; or x is 3 to 4 and each $R^3$ independently represents a $C_7$-$C_{18}$ alkyl group.

**5.** A compound according to any one of claims 1 to 4, wherein each of $R^4$, $R^5$ and $R^6$ represents a hydrogen atom and $R^7$ represents a group $-CH_2NHR^8$, or each of $R^4$ and $R^5$ represents a hydrogen atom and $R^6$ and $R^7$ together represent a group

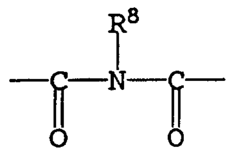

where $R^8$ is as defined in claim 1.

**6.** A compound according to any one of the preceding claims, wherein $-NHR^8$ or $-NR^8$ is derived from a primary amine of the general formula

$H_2N$-$(CHR^{11})_r$-$CH_2$-$[A$-$CH_2$-$(CHR^{11})_r]_s$-$NH_2$ (V)

wherein A is -NH or -O-, each $R^{11}$ independently represents a hydrogen atom or a methyl group, r is in the range 1 to 3, and s is in the range 1 to 8 when A is -NH or s is in the range 1 to 200 when A is -O-; or two groups $R^8$ together are a bridging group of the general formula

-$(CHR^{11})_r$-$CH_2$-$[A$-$CH_2$-$(CHR^{11})_r]_s$- (V')

where A, $R^{11}$, r and s are as defined above.

**7.** A process for the preparation of a compound of formula I as claimed in any one of claims 1 to 6 which comprises reacting a compound of the general formula

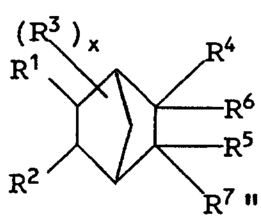

(VII)

wherein x, $R^1$ $R^2$, $R^3$, $R^4$, $R^5$ and $R^6$ are as defined in claim 1, provided that if a group $R^3$ represents a group of formula II, IIIA or IIIB then in formula IV $R^{7'}$ represents a group -CHO or -COOH or $R^6$ and $R^{7'}$ together represent a group

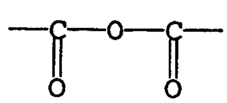

;

$R^{7''}$ represents a group -CHO or -COOH, or $R^6$ and $R^{7''}$ together represent a group

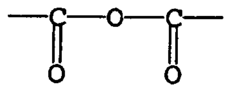 ;

with a compound of the general formula

B-H (VIII)

wherein, when $R^{7''}$ represents a -COOH group, then B represents a group X as defined above, or when $R^{7''}$ represents a -CHO group or when $R^6$ and $R^{7''}$ together represent a group

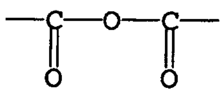 ;

then B represents a group $-NHR^8$ as defined above; and, where necessary or desired, selectively hydrogenating the product so obtained to reduce carbonyl (C = O) or imine (C = N) unsaturation.

**8.** An intermediate compound of formula VII as defined in claim 7.

**9.** A lubricating oil composition comprising a major amount of a lubricating oil and a minor amount of a compound of formula I as claimed in any one of claims 1 to 6.

**10.** A fuel composition comprising a major amount of a fuel and a minor amount of a compound of formula I as claimed in any one of claims 1 to 6.

**11.** An additive concentrate comprising an inert carrier fluid and from 10 to 80%w, based on the total concentrate, of a compound of formula I as claimed in any one of claims 1 to 6.

**12.** Use of a compound of formula I as claimed in any one of claims 1 to 6 as a dispersant additive.

European Patent Office

# EUROPEAN SEARCH REPORT

Application Number
EP 94 20 0495

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | US-A-3 873 276 (INSTITUT FRANCAIS DU PETROLE, DES CARBURANTS, ET LUBRIFIANTS)<br>* examples 17-20 * | 1,12 | C07D209/76<br>C10M133/16<br>C10M133/56<br>C10L1/22 |
| A | EP-A-0 393 769 (AGIP PETROLI S.P.A.)<br>* claims * | 1,12 | |
| A | EP-A-0 271 937 (SHELL INTERNATIONALE RESEARCH MAATSCHAPPIJ B.V.)<br>* claims * | 1,12 | |

TECHNICAL FIELDS SEARCHED (Int.Cl.5)

C07D
C10M
C10L

The present search report has been drawn up for all claims

| Place of search | Date of completion of the search | Examiner |
|---|---|---|
| THE HAGUE | 10 June 1994 | Van Bijlen, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document